# EUROPEAN PATENT SPECIFICATION

(11) **EP 2 950 778 B1**
(45) Date of publication and mention of the grant of the patent: **14.11.2018**
(21) Application number: 14701224.9
(22) Date of filing: 24.01.2014
(51) Int. Cl.: A61K 8/46, A61Q 5/02, A61K 8/60, A61Q 19/10, A61K 8/11, C11D 1/06, C11D 1/66, C11D 1/83, C11D 3/50, C11D 17/00, C11D 1/14

(54) **COMPOSITIONS WITH IMPROVED AESTHETIC AND SENSORIAL PROPERTIES**
ZUSAMMENSETZUNGEN MIT VERBESSERTEN ÄSTHETISCHEN UND SENSORISCHEN EIGENSCHAFTEN
COMPOSITIONS À PROPRIÉTÉS ESTHÉTIQUES ET SENSORIELLES AMÉLIORÉES

(30) Priority: 30.01.2013 EP 13153329
(43) Date of publication of application: 09.12.2015
(73) Proprietor: Unilever PLC, London, Greater London EC4Y 0DY (GB); Unilever N.V., 3013 AL Rotterdam (NL)
(72) Inventor: STEVENSON, Paul, Simon, Wirral Merseyside CH63 3JW (GB)
(74) Representative: McHugh, Paul Edward
(86) International application number: PCT/EP2014/051452
(87) International publication number: WO 2014/118095

(56) References cited:
- EP-A1- 1 445 302
- WO-A1-2011/051161
- DE-A1- 10 310 381
- DE-A1- 19 648 439
- US-A- 5 389 279
- US-A- 5 654 192
- US-A1- 2012 276 175
- US-B1- 6 362 146

## Description

_This invention relates to compositions for use in aqueous based treatments such as personal bathing, hand washing of fabrics and dishes, with improved aesthetic and sensorial properties in hard water.

In many regions, the water necessarily used in personal bathing or fabric/dish hand washing may be discoloured and contain a high level of undesirable calcium ions such that it is aesthetically displeasing to the eye. In some regions consumers would be willing to re-use 'grey' water or collected water. Such water may be safe to use for aqueous treatments but it is aesthetically displeasing and so off-putting.

EP 1 445 302 A1 discloses detergent compositions comprising at least one glycolipid biosurfactant and at least one non-glycolipid surfactant, whereby the glycolipid biosurfactant and the non-glycolipid surfactant are in the micellar phase.

An object of the invention is to provide a composition and a process for personal bathing and hand washing of dishes and/or fabrics with improved aesthetics in the context of low quality hard water.

According to the present invention there is provided a high foaming washing composition comprising (a) a surfactant combination comprising (i) a synthetic anionic surfactant; and (ii) a glycolipid biosurfactant which is present at a level of at least 25% of the total surfactant in said surfactant combination, and (b) one or more encapsulates comprising a sensorial benefit agent.

In a further aspect the present invention provides a high foaming personal or fabric or hard substrate wash liquor comprising water and:
(a) a surfactant combination comprising (i) a synthetic anionic surfactant; and (ii) a glycolipid biosurfactant which is present at a level of at least 25% of the total surfactant in said surfactant combination, and (b) one or more encapsulates comprising a sensorial benefit agent.

In a yet still further aspect the present invention provides a method of treating a substrate using a wash liquor comprising the steps of:
(i) in a receptacle such as a bath tub or bucket or sink, mixing with water a quantity of the composition of the first aspect of the invention to produce a high foaming wash liquor.

Preferably the method comprises the step of
(ii) applying a shear force to one or more of said encapsulates thereby releasing some or all of the sensorial benefit agent.

The combination of the invention synergistically provides an excellent aesthetic and sensory experience in less than ideal water conditions. High foam levels are provided conceal aesthetically displeasing (but albeit usable) liquor and this encourages the user to interact with the foamy wash liquor.

In the case of compositions/liquors also comprising a sensorial benefit agent, a further advantage is that this interaction releases the benefit agent and in turn enhances the sensorial experience. This encourages further interaction with the foamy wash liquor.

The substrate is preferably a fabric surface or a hard surface (such as a work surface or cutlery or crockery) or human skin or hair or teeth.

The invention is particularly advantageous where the reservoir is an open mouthed reservoir, such as a bathing tub or sink or bucket, and wherein the user must immerse their hands or other part of their body (also if only partially and/or whilst holding a washing tool such as scrubbing implement for a hard surface or e.g. tooth brush) into the wash liquor to carry out the washing operation either on the user's body or fabrics or hard surfaces.

Preferably the glycolipid comprises a rhamnolipid or a sophorolipid or any combination thereof. If sophorolipids are included, acidic forms of sophorolipids are preferred.

In the case of rhamnolipids, throughout this patent specification, the prefixes mono- and di- are used to indicate respectively to indicate mono-rhamnolipids (having a single rhamnose sugar ring) and di-rhamnolipids (having two rhamnose sugar rings) respectively. If abbreviations are used R1 is mono-rhamnolipid and R2 is di-rhamnolipid. Preferably the ratio of R1:R2 is such that R1 is always greater in proportion to R2.

Preferably the glycolipid is present at 25% -95% of the surfactant combination. More preferably the glycolipid is present at 50-75% of the surfactant combination.

The surfactant combination comprises a synthetic anionic surfactant. 'Anionic surfactants' are defined herein as amphiphilic molecules comprising one or more functional groups that exhibit a net anionic charge when in aqueous solution at the normal wash pH of between 4 and 11.

Preferred anionic surfactants are calcium intolerant.

Preferably the alkali metal salts of organic sulphur reaction products having in their molecular structure an alkyl moiety containing from about 6 to 24 carbon atoms, more greater than 12 carbon atoms and preferably also a moiety selected from the group consisting of sulphonic and sulphuric acid ester moieties. Additionally or alternatively, the anionic surfactant preferably has low levels of ethoxylation, preferably comprising 1-12 ethylene oxide units per molecule, more preferably 1-3 and even more preferably 1. The units of ethylene oxide may be an average.

Providing the formulation scientist with the freedom to use longer carbon chain lengths and/or lower levels of ethoxylation is greatly beneficial, not least on cost grounds. However these factors increase calcium intolerance and so such surfactants are advantageous selections for the present invention.

Although any anionic surfactant hereinafter described can be used, such as primary alkyl sulphates (PAS) e.g. sodium lauryl sulphate (SLS) and e.g. alkyl ether sulphate such as sodium lauryl ether sulphate(SLES), soaps, fatty acid ester sulphonates, fatty acid sulphates or sulphonates; alkyl benzene sulphonates (LAS), sulphosuccinate esters, olefin sulphonates, paraffin sulphonates and organic phosphates; fatty alcohol sulphates; alkyl phenol ether sulphate; fatty acyl isethionate products which products comprise fatty acyl isethionate and free fatty acid and/or fatty acid salt; alkyl sulphonates such as sodium alkane sulphonate. Preferred anionic surfactants are the alkali (ammonium or triethylammonium for example) and alkaline earth metal salts of the above. The source oil / alcohol can be plant or animal derived for example coconut or palm or tallow etc.

Preferred synthetic anionic surfactants are primary alkyl sulfates (PAS) or a Linear alkylbenzoate sulphonates (LAS).

The surfactant combination is present in the fabric or hard surface washing compositions at a level of from 3 to 85% by weight, preferably from 3 to 60% by weight, more preferably from 3 to 40% by weight, most preferably from 3 to 35% by weight.

The surfactant combination is present in personal (human skin and hair) wash compositions at a level of 5 to 60%, preferably 10 to 40% surfactant, while cosmetic compositions need not comprise any surfactant, but preferably comprise 1% to 30% by wt., more preferably 1 to 15% by wt. surfactant.

Preferred encapsulates comprise shear/pressure-sensitive action encapsulates, whereby the sensorial benefit agent is released in response to mechanical force (e.g., friction, pressure, shear stress) on the encapsulate.

Urea-formaldehyde and melamine-formaldehyde microcapsules may be used to provide the necessary friction or pressure based release mechanism.

Additionally or alternatively, the encapsulates may also be of diffusive action, wherein sensorial benefit agent is also released by diffusion through the outer wall of the encapsulate.

The encapsulate preferably comprises a shell or capsule surrounding a core, wherein the core comprises the sensorial benefit agent.

Preferably release of the sensorial benefit agent from the encapsulate is friction-based, the benefit becoming apparent after a rubbing process is applied to the encapsulate shell e.g. by rubbing between finger tips or between finger tips and the scalp, or between finger tips and a hard surface or fabric surface. Commercially available melamine formaldehyde based, friction release encapsulates are the Aroma Ball Type 1 and Aroma Ball S-series encapsulates ex. Polychrome, Korea.

Preferably the shell is a melamine formaldehyde shell.

The encapsulate shell is preferably comprised of materials including but not limited to polyurethane, polyamide, polyolefin, polysaccaharide, protein, silicone, lipid, modified cellulose, gums, polyacrylate, polyphosphate, polystyrene, polyesters or combinations of these materials. Other encapsulating material which may be used effectively in the present invention, such as polymethylmethacrylate. Preferred encapsulating polymers include those formed from melamine formaldehyde or urea formaldehyde condensates, as well as similar types of aminoplasts. Most preferably the shell comprises melamine formaldehyde.

Additionally, microcapsules made via the simple or complex coacervation of gelatin are suitable for use in compositions of the invention.

A representative process used for aminoplast encapsulation is disclosed in U.S. Patent No. 3,516,941 though it is recognised that many variations with regard to materials and process steps are possible. A representative process used for gelatin encapsulation is disclosed in U.S. Patent No, 2,800,457 though it is recognized that many variations with regard to materials and process steps are possible. Both of these processes are discussed in the context of fragrance encapsulation for use in consumer products in U.S. Patent Nos. 4,145,184 and 5,112,688 respectively.

Encapsulation can provide pore vacancies or interstitial openings depending on the encapsulation techniques employed.

Fragrance capsules known in the art and suitable for use in the present invention comprise a wall or shell comprising a three-dimensional cross-linked network of an aminoplast resin, more specifically a substituted or un-substituted acrylic acid polymer or co-polymer cross-linked with a urea-formaldehyde pre-condensate or a melamine-formaldehyde pre-condensate.

Microcapsule formation using mechanisms similar to the foregoing mechanism, using (i) melamine-formaldehyde or urea-formaldehyde pre-condensates and (ii) polymers containing substituted vinyl monomeric units having proton-donating functional group moieties (e.g. sulfonic acid groups or carboxylic acid anhydride groups) bonded thereto is disclosed in U.S. Patent 4,406,816 (2-acrylamido-2-methyl-propane sulfonic acid groups), published Patent Application GB 2,062,570 A (styrene sulfonic acid groups) and published Patent Application GB 2,006,709 A (carboxylic acid anhydride groups).

The encapsulate may further comprise a carrier oil in the core. The carrier oils are hydrophobic materials that are miscible in the volatile benefit agent materials used in the present invention. Suitable oils are those having reasonable affinity for the benefit agent. Where the benefit agent is a perfume, suitable materials include, but are not limited to triglyceride oil, mono and diglycerides, mineral oil, silicone oil, diethyl phthalate, polyalpha olefins, castor oil and isopropyl myristate. Preferably, the oil is a triglyceride oil, most preferably a capric/caprylic triglyceride oil.

Particle size and average diameter of the capsules can vary from about 10 nanometers to about 1000 microns, preferably from about 50 nanometers to about 100 microns, more preferably from about 2 to about 40 microns, even more preferably from about 4 to 15 microns. A particularly preferred range is from about 5 to 10 microns, for example 6 to 7 microns. The capsule distribution can be narrow, broad or multimodal. Multimodal distributions may be composed of different types of capsule chemistries.

The shell may further comprise a deposition aid, which is preferably covalently attached.

A preferred deposition aid is a polysaccharide. The polysaccharide preferably has a β-1,4-linked backbone.

Preferably the polysaccharide is a cellulose, a cellulose derivative, or another β-1,4-linked polysaccharide having an affinity for cellulose, such as polymannan, polyglucan, polyglucomannan, polyxyloglucan and polygalactomannan or a mixture thereof. More preferably, the polysaccharide is selected from the group consisting of polyxyloglucan and polygalactomannan.

Highly preferred polysaccharides are selected from locust bean gum, tamarind gum, xyloglucan, non-ionic guar gum, cationic starch and mixtures thereof. Most preferably, the deposition aid is locust bean gum.

Preferably, the polysaccharide backbone has only β-1,4 linkages. Optionally, the polysaccharide has linkages in addition to the β-1,4 linkages, such as β-1,3 linkages. Thus, optionally some other linkages are present. Polysaccharide backbones which include some material which is not a saccharide ring are also within the ambit of the present invention (whether terminal or within the polysaccharide chain).

The polysaccharide may be straight or branched. Many naturally occurring polysaccharides have at least some degree of branching, or at any rate at least some saccharide rings are in the form of pendant side groups (which are therefore not in themselves counted in determining the degree of substitution) on a main polysaccharide backbone.

Preferably, the polysaccharide is present at levels of between 0.1% to 10% w/w by weight of the total amount of the particle.

The deposition aid, which is preferably a polysaccharide, is attached to the particle by means of a covalent bond, entanglement or strong adsorption, preferably by a covalent bond or entanglement and most preferably by means of a covalent bond. By entanglement as used herein is meant that the deposition aid is adsorbed onto the particle as the polymerisation proceeds and the particle grows in size, part of the adsorbed deposition aid becomes buried within the interior of the particle. Hence at the end of the polymerisation, part of the deposition aid is entrapped and bound in the polymer matrix of the particle, whilst the remainder is free to extend into the aqueous phase.

By strong adsorption as used herein is meant strong adsorption of the deposition aid to the surface of the particle; such adsorption can, for example, occur due to hydrogen bonding, Van Der Waals or electrostatic attraction between the deposition aid and the particle.

The deposition aid is thus mainly attached to the particle surface and is not, to any significant extent, distributed throughout the internal bulk of the particle. This is distinct from graft copolymers in which e.g. a polysaccharide may be grafted along the length of a polymer chain. A particle which is formed from a graft copolymer would, therefore, contain polysaccharide throughout the internal bulk of the particle as well as on the particle surface and the present invention is not intended to cover such a particle. Thus the particle which is produced when using a polysaccharide as the deposition aid according to the process of the invention can be thought of as a "hairy particle", which is different from a graft copolymer. This feature of the invention provides significant cost reduction opportunities for the manufacturer as much less deposition aid is required to achieve the same level of activity as systems which utilise polysaccharide copolymers.

The deposition aid is present in the outermost portion of the shell, which is made of melamine formaldehyde polymer having a thickness of from 5 to 20 nm.

Polyesters of terephthalic and other aromatic dicarboxylic acids having soil release properties, in particular, the so-called PET/POET (polyethylene terephthalate/polyoxyethylene terephthalate) and PET/PEG (polyethylene terephthalate/polyethylene glycol) polyesters may be employed as deposition aids.

The polymer must have at least one mole free OH group per mole polymer, to allow covalent binding to the reactive dye(s). Most preferably the polymer comprises at least two free OH groups. Preferably the OH groups are the terminal groups of the polymer.

Preferably, the oxyalkyleneoxy [-O(CH₂)ₜO-] is selected from: oxy-1,2-propyleneoxy [-OCH₂CH(Me)O-]; oxy-1,3-propyleneoxy [O-CH₂CH₂CH₂O-]; and, oxy-1,2-ethyleneoxy [-OCH₂CH₂O-] (t is an interger). As is evident one or more of the CH₂ groups of the oxyalkyleneoxy may be substituted by C1 to C4 alkyl group(s).

The polyoxyalkyleneoxy facilitates water solubility of the polymer. Preferably, the polyoxyalkyleneoxy [-O(CH₂)_{w}-]ₛO- is selected from: polyoxy-1,2-propyleneoxy [-O(CH₂CH(Me)-]ₛO-; polyoxy-1,3-propyleneoxy [O-CH₂CH₂CH₂-]ₛO-; and, polyoxy-1,2-ethyleneoxy [O-CH₂CH₂-]ₛO-. The polyoxyalkyleneoxy may be a mixture of different oxyalkyleneoxy. Different polyoxyalkyleneoxy types may present in the polymer. (s and w are intergers).

Preferably the phenyl dicarboxylate is a 1,4-phenyl dicarboxylate. Preferably the phenyl dicarboxylate is of the form: -OC(O)C₆H₄C(O)O-.

Examples of preferred polymers are a PET/POET (Polyethylene terephthalate/polyoxyethylene terephthalate), PEG/POET (Polyethyleneglycol/ polyoxyethylene terephthalate) or PET/PEG (Polyethylene terephthalate/ Polyethyleneglycol) polymer. Most preferable a PET/POET.

The structure of a preferred polymer is found below. 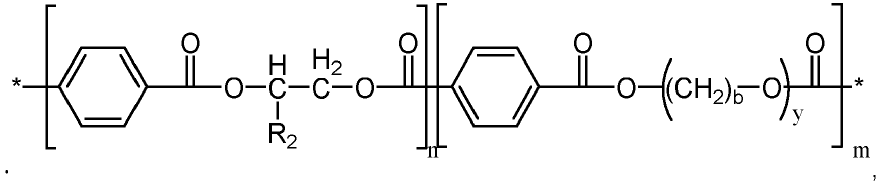 wherein
R₂ is selected from H or CH₃, preferably H;
b is 2 or 3, preferably 2;
y is 2 to 100, preferably 5 to 50;
n and m are independently 1 to 100, preferably 2 to 30; and, the terminal (end) groups of the polymer are (CH₂)_{b}OH.

The polymers may be synthesised by a variety of routes, for example an esterification reaction of dimethyl terephthalate with ethyleneglycol and polyethyleneglycol, this reaction is discussed in Polymer Bulletin 28, 451-458 (1992). Another example would be the direct esterification of terephthalic acid with ethylene glycol and/or propylene glycol and polypropylene glycol. A further example would be a transesterification of a polyethyleneterephthalate with a polyethyleneglycol or polypropylene gycol.

It is preferred that the number average molecular weight of the polymer is in the range from 1000 to 50,000, preferably the average molecular weight of the polymer is in the range of from 1000 to 15000, more preferably from 2000 to 10000.

Preferably the sensorial benefit agent comprises a skin benefit agent or an olfactory benefit agent and/or may be a volatile benefit agent. Sensorial benefit agents may also have benefits for hair and/or hard surfaces and/or fabrics. The sensorial benefit may have anti-foam properties, and as such it is advantageous for foaming purposes that it is encapsulated so as not interfering with the foam until release by rubbing.

Suitable volatile benefit agents include but are not limited to perfumes, insect repellents, essential oils, sensates such as menthol and aromatherapy actives, preferably perfumes. Mixtures of volatile benefit agents may be used.

The total amount of benefit agent is preferably from 0.01 to 10 % by weight, more preferably from 0.05 to 5 % by weight, even more preferably from 0.1 to 4.0 %, most preferably from 0.15 to 4.0 % by weight, based on the total weight of the composition.

The preferred benefit agent is a perfume. The compositions of the compositions of the invention also comprise an unconfined (also called non-encapsulated) volatile benefit agent. Where the volatile benefit agent is a perfume, the perfumes described below are suitable for use as the encapsulated volatile benefit agent and also as the unconfined perfume component.

Any suitable perfume or mixture of perfumes may be used. Useful components of the perfume include materials of both natural and synthetic origin. They include single compounds and mixtures. Specific examples of such components may be found in the current literature, e.g., in Fenaroli's Handbook of Flavor Ingredients, 1975, CRC Press; Synthetic Food Adjuncts, 1947 by M. B. Jacobs, edited by Van Nostrand; or Perfume and Flavor Chemicals by S. Arctander 1969, Montclair, N.J. (USA). These substances are well known to the person skilled in the art of perfuming, flavouring, and/or aromatizing consumer products, i.e., of imparting an odour and/or a flavour or taste to a consumer product traditionally perfumed or flavoured, or of modifying the odour and/or taste of said consumer product.

By perfume in this context is not only meant a fully formulated product fragrance, but also selected components of that fragrance, particularly those which are prone to loss, such as the so-called 'top notes'.

Top notes are defined by Poucher (Journal of the Society of Cosmetic Chemists 6(2):80 [1955]). Examples of well known top-notes include citrus oils, linalool, linalyl acetate, lavender, dihydromyrcenol, rose oxide and cis-3-hexanol. Top notes typically comprise 15-25%wt of a perfume composition and in those embodiments of the invention which contain an increased level of top-notes it is envisaged at that least 20%wt would be present within the encapsulate.

Some or all of the perfume or pro-fragrance may be encapsulated, typical perfume components which it is advantageous to encapsulate, include those with a relatively low boiling point, preferably those with a boiling point of less than 300, preferably 100-250 Celsius and pro-fragrances which can produce such components.

It is also advantageous to encapsulate perfume components which have a low Clog P (ie. those which will be partitioned into water), preferably with a Clog P of less than 3.0. These materials, of relatively low boiling point and relatively low Clog P have been called the "delayed blooming" perfume ingredients and include the following materials:
Allyl Caproate, Amyl Acetate, Amyl Propionate, Anisic Aldehyde, Anisole, Benzaldehyde, Benzyl Acetate, Benzyl Acetone, Benzyl Alcohol, Benzyl Formate, Benzyl Iso Valerate, Benzyl Propionate, Beta Gamma Hexenol, Camphor Gum, Laevo-Carvone, d-Carvone, Cinnamic Alcohol, Cinamyl Formate, Cis-Jasmone, cis-3-Hexenyl Acetate, Cuminic Alcohol, Cyclal C, Dimethyl Benzyl Carbinol, Dimethyl Benzyl Carbinol Acetate, Ethyl Acetate, Ethyl Aceto Acetate, Ethyl Amyl Ketone, Ethyl Benzoate, Ethyl Butyrate, Ethyl Hexyl Ketone, Ethyl Phenyl Acetate, Eucalyptol, Eugenol, Fenchyl Acetate, Flor Acetate (tricyclo Decenyl Acetate), Frutene (tricyclco Decenyl Propionate), Geraniol, Hexenol, Hexenyl Acetate, Hexyl Acetate, Hexyl Formate, Hydratropic Alcohol, Hydroxycitronellal, Indone, Isoamyl Alcohol, Iso Menthone, Isopulegyl Acetate, Isoquinolone, Ligustral, Linalool, Linalool Oxide, Linalyl Formate, Menthone, Menthyl Acetphenone, Methyl Amyl Ketone, Methyl Anthranilate, Methyl Benzoate, Methyl Benyl Acetate, Methyl Eugenol, Methyl Heptenone, Methyl Heptine Carbonate, Methyl Heptyl Ketone, Methyl Hexyl Ketone, Methyl Phenyl Carbinyl Acetate, Methyl Salicylate, Methyl-N-Methyl Anthranilate, Nerol, Octalactone, Octyl Alcohol, p-Cresol, p-Cresol Methyl Ether, p-Methoxy Acetophenone, p-Methyl Acetophenone, Phenoxy Ethanol, Phenyl Acetaldehyde, Phenyl Ethyl Acetate, Phenyl Ethyl Alcohol, Phenyl Ethyl Dimethyl Carbinol, Prenyl Acetate, Propyl Bornate, Pulegone, Rose Oxide, Safrole, 4-Terpinenol, Alpha-Terpinenol, and/or Viridine
Where non-encapsulated or 'free' perfume ingredients are used, preferred are those hydrophobic perfume components with a ClogP above 3. As used herein, the term "ClogP" means the calculated logarithm to base 10 of the octanol/water partition coefficient (P). The octanol/water partition coefficient of a perfume raw material (PRM) is the ratio between its equilibrium concentrations in octanol and water. Given that this measure is a ratio of the equilibrium concentration of a PRM in a non-polar solvent (octanol) with its concentration in a polar solvent (water), ClogP is also a measure of the hydrophobicity of a material-the higher the ClogP value, the more hydrophobic the material. ClogP values can be readily calculated from a program called "CLOGP" which is available from Daylight Chemical Information Systems Inc., Irvine Calif., USA. Octanol/water partition coefficients are described in more detail in U.S. Pat. No. 5,578,563.

Perfume components with a ClogP above 3 comprise: Iso E super, citronellol, Ethyl cinnamate, Bangalol, 2,4,6-Trimethylbenzaldehyde, Hexyl cinnamic aldehyde, 2,6-Dimethyl-2-heptanol, Diisobutylcarbinol, Ethyl salicylate, Phenethyl isobutyrate, Ethyl hexyl ketone, Propyl amyl ketone, Dibutyl ketone, Heptyl methyl ketone, 4,5-Dihydrotoluene, Caprylic aldehyde, Citral, Geranial, Isopropyl benzoate, Cyclohexanepropionic acid, Campholene aldehyde, Caprylic acid, Caprylic alcohol, Cuminaldehyde, 1-Ethyl-4-nitrobenzene, Heptyl formate, 4-Isopropylphenol, 2-Isopropylphenol, 3-Isopropylphenol, Allyl disulfide, 4-Methyl-1-phenyl-2-pentanone, 2-Propylfuran, Allyl caproate, Styrene, Isoeugenyl methyl ether, Indonaphthene, Diethyl suberate, L-Menthone, Menthone racemic, p-Cresyl isobutyrate, Butyl butyrate, Ethyl hexanoate, Propyl valerate, n-Pentyl propanoate, Hexyl acetate, Methyl heptanoate, trans-3,3,5-Trimethylcyclohexanol, 3,3,5-Trimethylcyclohexanol, Ethyl p-anisate, 2-Ethyl-1-hexanol, Benzyl isobutyrate, 2,5-Dimethylthiophene, Isobutyl 2-butenoate, Caprylnitrile, gamma-Nonalactone, Nerol, trans-Geraniol, 1-Vinylheptanol, Eucalyptol, 4-Terpinenol, Dihydrocarveol, Ethyl 2-methoxybenzoate, Ethyl cyclohexanecarboxylate, 2-Ethylhexanal, Ethyl amyl carbinol, 2-Octanol, 2-Octanol, Ethyl methylphenylglycidate, Diisobutyl ketone, Coumarone, Propyl isovalerate, Isobutyl butanoate, Isopentyl propanoate, 2-Ethylbutyl acetate, 6-Methyl-tetrahydroquinoline, Eugenyl methyl ether, Ethyl dihydrocinnamate, 3,5-Dimethoxytoluene, Toluene, Ethyl benzoate, n-Butyrophenone, alpha-Terpineol, Methyl 2-methylbenzoate, Methyl 4-methylbenzoate, Methyl 3, methylbenzoate, sec. Butyl n-butyrate, 1,4-Cineole, Fenchyl alcohol, Pinanol, cis-2-Pinanol, 2,4, Dimethylacetophenone, Isoeugenol, Safrole, Methyl 2-octynoate, o-Methylanisole, p-Cresyl methyl ether, Ethyl anthranilate, Linalool, Phenyl butyrate, Ethylene glycol dibutyrate, Diethyl phthalate, Phenyl mercaptan, Cumic alcohol, m-Toluquinoline, 6-Methylquinoline, Lepidine, 2-Ethylbenzaldehyde, 4-Ethylbenzaldehyde, o-Ethylphenol, p-Ethylphenol, m-Ethylphenol, (+)-Pulegone, 2,4-Dimethylbenzaldehyde, Isoxylaldehyde, Ethyl sorbate, Benzyl propionate, 1,3-Dimethylbutyl acetate, Isobutyl isobutanoate, 2,6-Xylenol, 2,4-Xylenol, 2,5-Xylenol, 3,5-Xylenol, Methyl cinnamate, Hexyl methyl ether, Benzyl ethyl ether, Methyl salicylate, Butyl propyl ketone, Ethyl amyl ketone, Hexyl methyl ketone, 2,3-Xylenol, 3,4, Xylenol, Cyclopentadenanolide and Phenyl ethyl 2 phenylacetate 2.

In the compositions of the present invention it is envisaged that there will be four or more, preferably five or more, more preferably six or more or even seven or more different perfume components from the list given of delayed blooming perfumes given above and/or the list of perfume components with a ClogP above 3 present in the perfume.

The sensorial benefit agent may also provide insect repellence in additional to providing a sensorial benefit. In chemical terms, most repellent actives belong to one of four groups: amides, alcohols, esters or ethers. Those suitable for use in the present invention are liquids or solids with a relatively low melting point and a boiling point above 150 °C, preferably liquids. They evaporate slowly at room temperature.

Advantageously the insect repellent is related to perfume species (most preferably the component falls into both classes). The most commonly used insect repellents include: DEET (N,N-diethyl-m-toluamide), essential oil of the lemon eucalyptus (Corymbia citriodora) and its active compound p-menthane-3,8-diol (PMD), Icaridin, also known as Picaridin, D-Limonene, Bayrepel, and KBR 3023, Nepetalactone, also known as "catnip oil", Citronella oil, Permethrin, Neem oil and Bog Myrtle.

Preferred insect repellents derived from natural sources include: Achillea alpina, alpha-terpinene, Basil oil (Ocimum basilicum), Callicarpa americana (Beautyberry), Camphor, Carvacrol, Castor oil (Ricinus communis), Catnip oil (Nepeta species), Cedar oil (Cedrus atlantica), Celery extract (Apium graveolens), Cinnamon (Cinnamomum Zeylanicum, leaf oil), Citronella oil (Cymbopogon fleusus), Clove oil (Eugenic caryophyllata), Eucalyptus oil (70%+ eucalyptol, also known as cineol), Fennel oil (Foeniculum vulgare), Garlic Oil (Allium sativum), Geranium oil (also known as Pelargonium graveolens), Lavender oil (Lavandula officinalis), Lemon eucalyptus (Corymbia citriodora) essential oil and its active ingredient p-menthane-3,8-diol (PMD), Lemongrass oil (Cymbopogon flexuosus), Marigolds (Tagetes species), Marjoram (Tetranychus urticae and Eutetranychus orientalis), Neem oil (Azadirachta indica), Oleic acid, Peppermint (Mentha x piperita), Pennyroyal (Mentha pulegium), Pyrethrum (from Chrysanthemum species, particularly C. cinerariifolium and C. coccineum), Rosemary oil (Rosmarinus officinalis), Spanish Flag Lantana camara (Helopeltis theivora), Solanum villosum berry juice, Tea tree oil (Melaleuca alternifolia) and Thyme (Thymus species) and mixtures thereof.

Preferred encapsulated insect repellents are mosquito repellents available from Celessence, Rochester, England. Celessence Repel, containing the active ingredient Saltidin™ and Celessence Repel Natural, containing the active Citrepel™ 75. Saltidin is a man made molecule developed originally by the Bayer Corporation. Citrepel is produced from eucalyptus oils and is high in p-menthane-3,8-diol (PMD). A preferred non-encapsulated repellent is Citriodiol™ supplied by Citrefine.

Preferably the sensory benefit agent comprises so-called 'aromatherapy' materials. These include components of essential oils such as Clary Sage, Eucalyptus, Geranium, Lavender, Mace Extract, Neroli, Nutmeg, Spearmint, Sweet Violet Leaf and Valerian.

Other sensorial benefit agents for use in this invention are meant to include but not be limited to moisturizers and/or emollients for skin and/or hair.

Oil/emollient particles (particularly when compositions are in lamellar phase) may be as set forth below:
Vegetable oils: Arachis oil, cannola oil, castor oil, cocoa butter, coconut oil, corn oil, cotton seed oil, olive oil, palm kernel oil, rapeseed oil, safflower seed oil, sesame seed oil and soybean oil.

Esters: Butyl myristate, cetyl palmitate, decyloleate, glyceryl laurate, glyceryl ricinoleate, glyceryl stearate, glyceryl isostearate, hexyl laurate, isobutyl palmitate, isocetyl stearate, isopropyl isostearate, isopropyl laurate, isopropyl linoleate, isopropyl myristate, isopropyl palmitate, isopropyl stearate, propylene glycol monolaurate, propylene glycol ricinoleate, propylene glycol stearate, and propylene glycol isostearate.

Animal Fats: Acytylatelte lanolin alcohols, lanolin, lard, mink oil and tallow.

Fatty acids and alcohols: Behenic acid, palmitic acid, stearic acid, behenyl alcohol, cetyl alcohol, eicosanyl alcohol and isocetyl alcohol.

Other examples of oil/emollients include mineral oil, petrolatum, silicone oil such as dimethyl polysiloxane, lauryl and myristyl lactate.

Additional emollient/oil generally will comprise, if present, 1% to 20% of the composition.

The composition is especially useful for washing in water with a high water hardness, preferably of greater than 5°FH preferably greater than 40°FH, more preferably greater than 90°FH.

The compositions of the invention are preferably unbuilt.

The compositions of the invention may comprise other ingredients as described hereinbelow.

Hand washing and fabric cleaning compositions may comprise polyester substantive soil release polymers, hydrotropes, opacifiers, colorants, other enzymes, further surfactants such as nonionic, cationic and or amphoteric surfactants, microcapsules of ingredients such as perfume or care additives, softeners, polymers for anti redeposition of soil, bleach, bleach activators and bleach catalysts, antioxidants, pH control agents and buffers, thickeners, external structurants for rheology modification, visual cues, either with or without functional ingredients embedded therein and other ingredients known to those skilled in the art.

The composition is preferably a liquid, gel but may also be a free flowing particulate, paste, or tabletted.

The invention will be further described with reference to the following non-limiting examples.

**1. Example Laundry Forumulation**

| **Ingredient** | **Liquid Fabric Washing Formulation** (concentration in final product [weight %]) |
|---|---|
| linear alkyl benzene sulfonate | 14 |
| Rhamnolipid JBR425 ex Jeneil | 14 |
| enzymes | 3 |
| MPG | 20 |
| TEA | 2 |
| sodium citrate | 2 |
| Sodium chloride | 2 |
| sodium hydroxide | 1 |
| dispersent (e.g. polycarboxylate) | 1 |
| Friction based melamine formaldehyde based encapsulates: Aroma Ball Type 1" and Aroma Ball S-series ex Polychrom | 0.1 |
| water & foam control & other minors | Balance to 100 |

**2. Example Personal Wash Composition**

| Material Name | Concentration in final product % by weight |
|---|---|
| Texapon N701 | 6 |
| Biosurfactant (JBR425) | 6 |
| Water | 38.5 |
| Carbopol 980 | 0.4 |
| Friction based melamine formaldehyde based encapsulates: Aroma Ball Type 1" and Aroma Ball S-series ex Polychrom S-series | 0.7 |
| Jaguar C14S | 0.2 |
| DC7051 HS | 0.75 |
| Tergobetaine CK | 1.6 |
| Glydant Plus | 0.1 |
| NaOH | 0.1 |
| NaCl | 1 |

### Method to produce rhamnolipd in varying fractions of R1 and R2.

JBR425 was acidified to pH 3 using 12M HCI and placed in a refrigerator overnight. The supernatant was then extracted three times using a 2:1 mixture of Chloroform and Ethanol. The solvent was then removed by rotary evaporation and the isolated rhamnolipid mixture was then re-dissolved in methanol.

The process of separating and characterising the mixture was carried out using an HPLC connected to an Ion Trap Electrospray ionisation Mass Spectrometer. The mode of ionisation was in negative mode with a scanning range of 50-1200Da. The column used to separate was a Phenomenex luna C18 250 x 4.6mm 5 µm column. The mobile phase: water (mobile phase A) and acetonitrile (mobile phase B) were used to separate via a gradient of 60:40 (A:B) changing to 30:70 (A:B) over 30 minutes. The system was then held for 5 minutes before returning to the start conditions all at a flow rate of 0.5ml/min. The injection volume was 10 µl.

### Foaming Test.

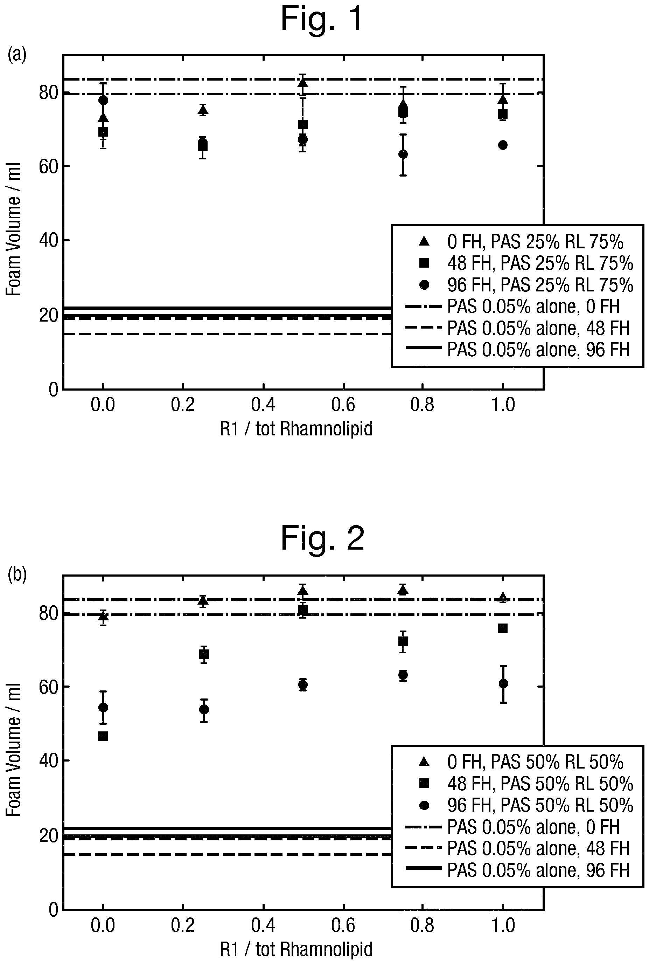

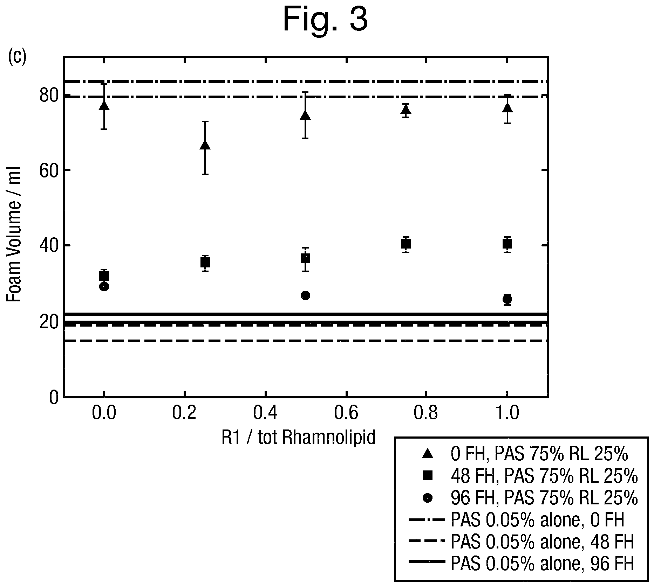

Increasing fractions of PAS (Galaxy Surfactants Ltd., Mumbai) were substituted with a rhamnolipid mixture of mono- (R1) and di- (R2) rhamnolipid (a: 75% RL - shown in figure 1; b: 50% RL - shown in figure 2 ; c: 25% RL - shown in figure 3). In the x-axis of the plots of figures 1,2 and 3, 0.0 corresponds to pure R2 and 1.0 to pure R1. The complete results set is shown in Table 1.

Method for Measuring Foaming of Biosurfactant Formualtions:
- A stock solution containing 0.5gpl total surfactant solution at the required FH by addition of dilute Calcium Chloride is made up
- the pH to pH 8 is checked and adjusted using MEA
- 30ml of the surfactant solution is measured out into a clean 100ml measuring cylinder.
- A standard Ross miles testing is carried out by inverting the cylinder 20 times and measuring the total volume after inversion.
- Measurements carried out in triplicate and averaged

The overall surfactant combination (total surfactant) concentration of the wash liquor was 0.05%.

**Table 1L: Foam volume from biosurfactant solutions as a function of level of calcium**

| Surfactant Composition | | | | | | | | |
|---|---|---|---|---|---|---|---|---|
| | 0.5gpl | | OFH | | 48FH | | 96FH | |
| % R1 | % R2 | %PAS | Foam Height/ml | Std Dev | Foam Height/ml | Std Dev | Foam Height/ml | Std Dev |
| 0 | 0 | 100 | 110 | 2 | 46 | 2 | 50 | 1 |
| 0 | 75 | 25 | 102 | 5.6 | 98 | 4.2 | 106.5 | 4.9 |
| 18.75 | 56.25 | 25 | 104 | 1.4 | 94 | 2.8 | 95 | 1.4 |
| 37.5 | 37.5 | 25 | 111 | 2.8 | 100 | 7 | 96 | 1.4 |
| 56.25 | 18.75 | 25 | 105.5 | 4.9 | 103.5 | 0.7 | 92 | 5.6 |
| 75 | 0 | 25 | 106.5 | 4.9 | 103 | 1.4 | 94.5 | 0.7 |
| 0 | 50 | 50 | 107.5 | 2.1 | 75.5 | 0.7 | 83.3 | 4.1 |
| 12.5 | 37.5 | 50 | 112 | 1.4 | 97.5 | 2.1 | 82.6 | 3 |
| 25 | 25 | 50 | 114.5 | 2.1 | 109.5 | 2.1 | 89.3 | 1.5 |
| 37.5 | 12.5 | 50 | 115 | 1.4 | 101 | 2.8 | 92 | 1 |
| 50 | 0 | 50 | 112.5 | 0.7 | 104.5 | 0.7 | 89.6 | 4.7 |
| 0 | 25 | 75 | 105.6 | 5.8 | 60.6 | 2 | 58 | 1.4 |
| 7.5 | 17.5 | 75 | 95 | 7 | 64.3 | 2 | na | na |
| 12.5 | 12.5 | 75 | 103.3 | 6 | 65.3 | 3.2 | 55.5 | 3.5 |
| 17.5 | 7.5 | 75 | 104.6 | 1.5 | 69.3 | 2 | na | na |
| 25 | 0 | 75 | 105 | 3.6 | 69.3 | 2 | 54.5 | 0.7 |

The data shows that:
- Both R1 and R2 forms of rhamnolipid maintain the foaming performances of the PAS in absence of Ca²⁺ ions (0 FH).
- Both R1 and R2 improve the foaming of PAS solutions up to 96 FH (results for PAS are shown as baseline).
- R1 performs better than R2 in boosting foam in presence of Ca²⁺.

## Claims

1. A high foaming washing composition comprising (a) a surfactant combination comprising (i) a synthetic anionic surfactant; and (ii) a glycolipid biosurfactant which is present at a level of at least 25 % of the total surfactant in said surfactant combination, and (b) one or more encapsulates comprising a sensorial benefit agent.

2. A high foaming washing composition according to claim 1 wherein the glycolipid comprises a rhamnolipid or a sophorolipid or any combination thereof.

3. A high foaming washing composition according to any preceding claim wherein the glycolipid comprises a rhamnolipid in which the ratio of mono-rhamnolipid (R1) : di-rhamnolipid (R2) is such that R1 is always greater in proportion to R2.

4. A high foaming washing composition according to any preceding claim wherein the glycolipid is present at 25% -95% of the surfactant combination.

5. A high foaming washing composition according to any preceding claim wherein the synthetic anionic surfactant is a primary alkyl sulfate (PAS) or a Linear alkylbenzoate sulphonate (LAS).

6. A high foaming washing composition according to any preceding claim wherein the sensorial benefit agent is a volatile benefit agent.

7. A high foaming washing composition according to any preceding claim wherein the sensorial benefit agent comprises a skin benefit agent or an olfactory benefit agent

8. A high foaming washing composition according to any preceding claim wherein the sensorial benefit agent comprises a perfume or comprises moisturizers and/or emollients.

9. A high foaming washing composition according to any preceding claim wherein the encapsulate release is friction-based, the benefit becoming apparent after a rubbing process is applied to the encapsulate shell.

10. A high foaming personal or fabric or hard substrate wash liquor comprising a mixture of water and the composition according to any preceding claim.

11. A method of treating a substrate using a wash liquor comprising the steps of:
in a receptacle such as a bath tub or bucket or sink, mixing with water a quantity of the composition of any of claims 1-9 to produce a high foaming wash liquor.

12. A method according to claim 11 further comprising the step of applying a shear force to one or more of said encapsulates thereby releasing some or all of said sensorial benefit agent.

13. A method according to claim 12 wherein the substrate is a fabric surface or a hard surface or human skin or hair or teeth.

14. A method according to claim 13 wherein the hard surface is a work surface or cutlery or crockery.

15. A method according to claim 12, 13 or 14 wherein the reservoir is an open mouthed reservoir, such as a bathing tub or sink or bucket, and wherein the user immerses a part of the body into the wash liquor to carry out the washing operation either on the user's body and/or fabrics and/or a hard surface.

## Patentansprüche

1. Stark schäumende Waschmittelzusammensetzung, umfassend (a) eine Tensidkombination, umfassend (i) ein synthetisches anionisches Tensid und (ii) ein Glycolipid-Biotensid, das mit einer Konzentration von mindestens 25% des gesamten Tensids in der Tensidkombination vorliegt, und (b) ein oder mehrere Einkapselungen, die ein sensorisches Vorteilsmittel umfassen.

2. Stark schäumende Waschmittelzusammensetzung nach Anspruch 1, wobei das Glycolipid ein Rhamnolipid oder ein Sophorolipid oder irgendeine Kombination davon umfasst.

3. Stark schäumende Waschmittelzusammensetzung nach irgendeinem vorhergehenden Anspruch, wobei das Glycolipid ein Rhamnolipid umfasst, wobei das Verhältnis von Mono-Rhamnolipid (R1):Di-Rhamnolipid (R2) derartig ist, dass R1 im Verhältnis zu R2 stets größer ist.

4. Stark schäumende Waschmittelzusammensetzung nach irgendeinem vorhergehenden Anspruch, wobei das Glycolipid mit 25%-95% der Tensidkombination vorliegt.

5. Stark schäumende Waschmittelzusammensetzung nach irgendeinem vorhergehenden Anspruch, wobei das synthetische anionische Tensid ein primäres Alkylsulfat (PAS) oder ein lineares Alkylbenzoatsulfonat (LAS) ist.

6. Stark schäumende Waschmittelzusammensetzung nach irgendeinem vorhergehenden Anspruch, wobei das sensorische Vorteilsmittel ein flüchtiges Vorteilsmittel ist.

7. Stark schäumende Waschmittelzusammensetzung nach irgendeinem vorhergehenden Anspruch, wobei das sensorische Vorteilsmittel ein Hautvorteilsmittel oder ein Geruchsvorteilsmittel ist.

8. Stark schäumende Waschmittelzusammensetzung nach irgendeinem vorhergehenden Anspruch, wobei das sensorische Vorteilsmittel ein Parfüm umfasst oder Befeuchtungsmittel und/oder Erweichungsmittel umfasst.

9. Stark schäumende Waschmittelzusammensetzung nach irgendeinem vorhergehenden Anspruch, wobei die Einkapselungsfreisetzung auf Reibung beruht, wobei der Vorteil offensichtlich wird, nachdem ein Reibungsvorgang auf die Einkapselungshülle angewandt wurde.

10. Stark schäumende Waschlauge für ein Körperpflege- oder textiles- oder hartes Substrat, umfassend eine Mischung aus Wasser und der Zusammensetzung nach irgendeinem vorhergehenden Anspruch.

11. Verfahren zum Behandeln eines Substrats unter Verwendung einer Waschlauge, umfassend die Schritte: Mischen von Wasser mit einer Menge der Zusammensetzung nach irgendeinem der Ansprüche 1 bis 9 in einem Behälter, wie in einer Badewanne oder einem Eimer oder einem Spülbecken, um eine stark schäumende Waschlauge herzustellen.

12. Verfahren nach Anspruch 11, ferner umfassend den Schritt des Anwendens einer Scherkraft auf eine oder mehrere Einkapselungen, wobei das sensorische Vorteilsmittel teilweise oder gänzlich freigesetzt wird.

13. Verfahren nach Anspruch 12, wobei das Substrat eine textile Oberfläche oder eine harte Oberfläche oder menschliche(s) Haut oder Haar oder Zähne darstellt.

14. Verfahren nach Anspruch 13, wobei die harte Oberfläche eine Arbeitsfläche oder Besteck oder Geschirr ist.

15. Verfahren nach Anspruch 12, 13 oder 14, wobei das Reservoir ein offenes Reservoir ist, wie eine Badewanne oder ein Spülbecken oder ein Eimer, und wobei der Benutzer einen Teil des Körpers in die Waschlauge eintaucht, um den Waschvorgang entweder auf dem Körper des Benutzers und/oder den textilen Erzeugnissen und/oder einer harten Oberfläche auszuführen.

## Revendications

1. Composition de lavage moussant fortement comprenant (a) une combinaison de tensioactifs comprenant (i) un tensioactif anionique synthétique ; et (ii) un biotensioactif de type glycolipide qui est présent à raison d'au moins 25 % du tensioactif total dans ladite combinaison de tensioactifs, et (b) un ou plusieurs agents d'encapsulation comprenant un agent sensoriel bénéfique.

2. Composition de lavage moussant fortement selon la revendication 1, dans laquelle le glycolipide comprend un rhamnolipide ou un sophorolipide ou l'une quelconque de leurs combinaisons.

3. Composition de lavage moussant fortement selon l'une quelconque des revendications précédentes, dans laquelle le glycolipide comprend un rhamnolipide dans lequel le rapport du mono-rhamnolipide (R1) au di-rhamnolipide (R2) est tel que la proportion de R1 soit toujours supérieure à celle de R2.

4. Composition de lavage moussant fortement selon l'une quelconque des revendications précédentes, dans laquelle le glycolipide est présent à raison de 25 % à 95 % de la combinaison de tensioactifs.

5. Composition de lavage moussant fortement selon l'une quelconque des revendications précédentes, dans laquelle le tensioactif anionique synthétique est un alkylsulfate primaire (PAS) ou un alkylbenzoate-sulfonate linéaire (LAS).

6. Composition de lavage moussant fortement selon l'une quelconque des revendications précédentes, dans laquelle l'agent sensoriel bénéfique est un agent bénéfique volatil.

7. Composition de lavage moussant fortement selon l'une quelconque des revendications précédentes, dans laquelle l'agent sensoriel bénéfique comprend un agent bénéfique pour la peau ou un agent olfactif bénéfique.

8. Composition de lavage moussant fortement selon l'une quelconque des revendications précédentes, dans laquelle l'agent sensoriel bénéfique comprend un parfum ou comprend des agents hydratants et/ou émollients.

9. Composition de lavage moussant fortement selon l'une quelconque des revendications précédentes, dans laquelle la libération de l'agent d'encapsulation se base sur le frottement, le bénéfice devenant évident après qu'un processus de frottement a été appliqué à la coquille d'agent d'encapsulation.

10. Liqueur de lavage pour substrat dur ou pour étoffes ou pour l'hygiène personnelle moussant fortement comprenant un mélange d'eau et de la composition selon l'une quelconque des revendications précédentes.

11. Procédé pour traiter un substrat utilisant une liqueur de lavage, comprenant les étapes suivantes : dans un réceptacle tel qu'une baignoire ou un seau ou un évier, le mélange avec de l'eau d'une quantité de la composition de l'une quelconque des revendications 1 à 9 pour produire une liqueur de lavage moussant fortement.

12. Procédé selon la revendication 11, comprenant en outre l'étape d'application d'une force de cisaillement à un ou plusieurs desdits agents d'encapsulation, en libérant ainsi tout ou partie dudit agent sensoriel bénéfique.

13. Procédé selon la revendication 12, dans lequel le substrat est un substrat en étoffe ou une surface dure ou la peau ou les cheveux ou les dents d'un humain.

14. Procédé selon la revendication 13, dans lequel la surface dure est une surface de travail ou un couvert ou de la vaisselle.

15. Procédé selon la revendication 12, 13 ou 14, dans lequel le réservoir est un réservoir à goulot ouvert, tel qu'une baignoire ou un seau ou un évier, et dans lequel l'utilisateur immerge une partie du corps dans la liqueur de lavage pour réaliser l'opération de lavage sur le corps de l'utilisateur et/ou sur des étoffes et/ou sur une surface dure.
